# EUROPÄISCHE PATENTANMELDUNG

(11) **EP 2 848 232 A1**
(43) Veröffentlichungstag der Anmeldung: **18.03.2015**
(21) Anmeldenummer: 13184743.6
(22) Anmeldetag: 17.09.2013
(51) Int. Cl.: A61F 2/915, A61F 2/06

(54) **Stent mit Strömungsleitelementen**

(71) Anmelder: Cortronik GmbH, 18119 Rostock-Warnemünde (DE)
(72) Erfinder: Stiehm, Michael, 18057 Rostock (DE); Lootz, Daniel, 18119 Rostock (DE); Brede Martin, 18059 Rostock (DE); Leder, Alfred, 18209 Wittenbeck (DE)
(74) Vertreter: Galander, Marcus

(57) **Zusammenfassung**

Die Erfindung beschreibt einen Stent 1 mit Strömungsleitelementen 3a, 3b und 3c, die eine größere Höhe als die Grundelemente 5 aufweisen. Dadurch wird die Strömung durch den Stent 1 in Form einer Schraube geführt.

## Beschreibung

Die vorliegende Erfindung betrifft eine intraluminale Endoprothese geeignet zur Implantation in einem durchströmten Körpergefäß und aufweisend einen zylindrischen Grundkörper mit einem ersten Ende und einem zweiten Ende, wobei der Grundkörper aus einem Grundgerüst von zumindest zwei einzelnen, miteinander verbundenen Elementen besteht, und das Grundgerüst geeignet ist, eine mechanische Stützwirkung im Körpergefäß zu erzielen.

Unter einer intraluminalen Endoprothese werden im Rahmen dieser Erfindung alle Implantate verstanden, die zur Implantation in ein Körpergefäß geeignet sind. Dies sind insbesondere Stents, die zumeist mittels Kathetertechnik in Körpergefäße (Hohlorgane, insbesondere Blutgefäße) eingebracht werden. Derartige Endoprothesen werden insbesondere in die Körpergefäße implantiert, um diese offen zu halten. Dies können beispielsweise Blutgefäße nach einer Angioplastie sein. Des Weiteren werden derartige Endoprothesen auch in der Krebsbehandlung angewandt, um Atemwege, Gallenwege oder die Speiseröhre nach Verengung durch bösartige Tumore offen zu halten.

Im Folgenden wird die Erfindung anhand eines Stents beschrieben, wie er bei der Behandlung von Verengungen in arteriellen Blutgefäßen verwendet wird. Die vorliegende Erfindung ist für diese Anwendung besonders geeignet, jedoch nicht darauf eingeschränkt.

Die als Stenosen bezeichneten Verengungen von arteriellen Blutgefäßen werden zumeist durch Arteriosklerose verursacht. Dabei lagern sich Blutfette, Thromben, Bindegewebe und in geringen Mengen auch Kalk in den Gefäßwänden ab. Diese verhärten und die Blutgefäße verengen. Derartige Verengungen können mit einer sogenannten Angioplastie behandelt werden. Dabei wird mittels Kathetertechnik ein Ballon an die Stelle der Verengung geführt, mit einem Fluid beaufschlagt, aufgedehnt und so die Verengung wieder erweitert. Um das Gefäß dauerhaft offen zu halten, wird an die Stelle der Verengung ein Stent implantiert.

Ein derartiger Stent besteht aus einem im Wesentlichen zylindrischen und durchströmbaren Grundkörper. Dieser Grundkörper wird durch ein Grundgerüst gebildet, welches eine offene Struktur hat und geeignet ist, eine mechanische Stützwirkung auf das Gefäß auszuüben. Das Grundgerüst besteht dabei aus mehreren, mindestens zwei, einzelnen Elementen, die miteinander verbunden sind.

Unter dem Grundgerüst einer intraluminalen Endoprothese wird im Rahmen der Erfindung eine offene Struktur verstanden, die eine mechanische Stützwirkung auf das Körpergefäß ausübt.

Der vorliegenden Erfindung liegt die Aufgabe zugrunde, eine intraluminale Endoprothese, insbesondere einen Stent oder einen Stentgraft, derart auszugestalten, dass sich in der durchströmten Endoprothese eine möglichst natürliche Strömung ausbildet.

Die gestellte Aufgabe wird durch eine intraluminale Endoprothese mit den Merkmalen des unabhängigen Anspruches 1 gelöst. Vorteilhafte Ausgestaltungen der Erfindung werden in den abhängigen Ansprüchen aufgeführt.

Erfindungsgemäß weist das Grundgerüst Mittel auf, die zur Strömungsführung innerhalb des Grundkörpers geeignet sind, wenn der Grundkörper von seinem ersten Ende zu seinem zweiten Ende durchströmt wird, und die Mittel derart ausgestaltet sind, dass die Strömung innerhalb des Grundkörpers als Schraube geführt wird.

Der Erfindung liegt die Erkenntnis zugrunde, dass sich in einem natürlichen Gefäß eine spiral- oder schraubenförmige Strömung ausbildet. Dies gilt insbesondere in arteriellen Blutgefäßen. Die erfindungsgemäßen Endoprothesen weisen entsprechend Mittel auf, die zur Führung der Strömung als Schraube geeignet sind, wenn die Endoprothese von ihrem ersten zum zweiten Ende durchströmt wird.

Unter einer Schraube (oder Helix, diese Begriffe werden im Rahmen dieser Anmeldung synonym verwendet) wird im Rahmen dieser Erfindung eine dreidimensionale Kurve mit einer Steigung verstanden, die sich um den Mantel eines Zylinders windet. Entsprechend übertragen auf die Endoprothese folgt eine schraubenförmige Strömung einer dreidimensionalen Kurve mit einer Steigung, die sich entlang des Zylindermantels windet, welcher sich durch den Grundkörper der Endoprothese ergibt.

Die Mittel zur Strömungsführung sind dabei erfindungsgemäß derart ausgestaltet, dass die Strömung als Schraube geführt wird, wenn die Endoprothese in das Körpergefäß implantiert und durchströmt wird. Die Mittel zur Strömungsführung sind so ausgestaltet, dass sich eine schraubenförmige Strömungsführung ergibt, wenn die zylindrische Form des Grundkörpers, gebildet durch das Grundgerüst, bei der Implantation erhalten bleibt. Mit anderen Worten, die Mittel zur Strömungsführung als Schraube werden für die Durchströmung des zylindrischen Grundgerüstes ausgestaltet. Endoprothesen, die derart ausgestaltet sind, aber bei Implantation durch den Implantationsort ihre Form beispielsweise in einen eher kegelstumpfförmigen Grundkörper ändern, so dass sich am Implantationsort bei Durchströmung eine spiralförmige Strömung ergibt, werden von der vorliegenden Erfindung mit umfasst.

Die vorliegende Erfindung ist insbesondere für Stents geeignet, die in ein arterielles Körpergefäß implantiert werden. In arteriellen Gefäßen herrscht eine schraubenförmige Strömung. Ein erfindungsgemäßer Stent stellt sicher, dass auch nach Angioplastie und Implantation des Stents wie eingangs beschrieben weiter eine natürliche und schraubenförmige Durchströmung des Körpergefäßes mit Stent aufrecht erhalten bleibt. Im Köpergefäß ohne Implantat (Stent) wird durch die schraubenförmige Strömung des Blutes eine Scherspannung auf die Gefäßwand ausgeübt. Diese Scherspannung wird durch den erfindungsgemäßen Stent aufrecht erhalten. Durch die Scherspannung wird ein übermäßiges Wachstum von Endothelzellen nach Implantation des Stents verhindert, wodurch die Gefahr der erneuten Verengung (Restenose) des Gefäßes reduziert wird. Gemäß einer bevorzugten Ausgestaltung der Erfindung besteht das Grundgerüst aus zumindest zwei Grundelementen und zumindest zwei Strömungsleitelementen, wobei zumindest eine erste Gruppe von zumindest zwei Strömungsleitelementen derart angeordnet ist, dass die Strömungsleitelemente der der ersten Gruppe einer dreidimensionalen Kurve in Form einer Schraube folgen.

In dieser Ausgestaltung der Erfindung setzt sich das Grundgerüst aus mehreren Elementen zusammen, wobei nicht alle Elemente zur Führung der Strömung beitragen. Das aus den einzelnen Elementen bestehende Grundgerüst hat im Wesentlichen die Funktion, eine mechanische Stützwirkung auf das Körpergefäß auszuüben. Wenn die Endoprothese ein Stent ist, soll wie eingangs geschildert, die mittels Angioplastie erreichte Aufweitung des Körpergefäßes durch den Stent aufrecht erhalten werden. In dieser Ausgestaltung der Erfindung besteht das Grundgerüst aus Elementen, die nur dem Erreichen der Stützwirkung dienen, den Grundelementen, und aus Elementen, die zur Führung der Strömung dienen, den Strömungsleitelementen. Die Strömungsleitelemente können dabei auch zur mechanischen Stützwirkung der Endoprothese beitragen. Dies ist aber nicht erforderlich, entscheidend ist ihr Einfluss zur Führung der Strömung. Die Grundelemente haben keinen oder nur einen vernachlässigbaren Einfluss auf die Führung der Strömung, wenn die Endoprothese von ihrem ersten zu ihrem zweiten Ende durchströmt wird. Die Strömungsleitelemente sind in dieser Ausgestaltung der Erfindung derart angeordnet, dass sie dem dreidimensionalen Kurvenverlauf einer Schraube folgen. Die Strömung folgt in dieser Ausgestaltung der Erfindung den Verlauf der Strömungsleitelemente bzw. das durchströmende Medium bewegt sich entlang der Strömungsleitelement. Dadurch ergibt sich automatisch eine schraubenförmige Führung der Strömung, wenn die Endoprothese von ihrem ersten Ende in Richtung ihres zweiten Endes durchströmt wird.

Besonders bevorzugt weist das Grundgerüst mehrere Gruppen von Strömungsleitelementen auf, wobei die Strömungsleitelement in jeder Gruppe so angeordnet sind, dass sie einer dreidimensionalen Kurve in Form einer Schraube folgen, wobei Radius, Steigung und Gängigkeit der Schraube für jede Gruppe von Strömungsleitelementen identisch ist. In dieser bevorzugten Ausgestaltung der Erfindung werden die Strömungsleitelemente in Gruppen derart angeordnet, dass jede Gruppe dem dreidimensionalen Kurvenverlauf einer Schraube folgt. Die charakteristischen Merkmale der Schraube, Radius, Steigung und Gängigkeit, sind für alle Gruppen von Strömungsleitelementen gleich. Die Strömungsleitelemente einer Gruppe folgen so dem Verlauf einer dreidimensionalen Schraube. Die Strömungsleitelemente der nächsten Gruppe folgen dem Verlauf der gleichen dreidimensionalen Schraube, die jedoch gegenüber der Schraube der ersten Gruppe der Strömungsleitelemente verschoben ist. Gleiches gilt für die weiteren Gruppen von Strömungsleitelementen. Damit weist die Endoprothese dieser bevorzugten Ausgestaltung der Erfindung mehrere Gruppen von Strömungsleitelementen in mehreren gleichartigen Schrauben auf, entlang derer die Strömung durch die Endoprothese geführt wird.

Vorteilhafterweise besteht das Grundgerüst aus mehreren ringförmigen Segmenten, wobei jedes Segment aus mindestens einem Grundelemente und mindestens einem Strömungsleitelement besteht, und die Segmente über mindestens einen Verbinder miteinander verbunden sind. Dabei weist das Grundgerüst zweckmäßigerweise mindestens zwei ringförmige Segmente auf, die parallel zueinander oder gespiegelt zueinander angeordnet sind.

In diesen Ausgestaltungen der Erfindung besteht das Grundgerüst aus ringförmigen Segmenten, die über mindestens einen Verbinder miteinander verbunden sind. Die Segmente wiederum bestehen aus mindestens einem, bevorzugt mehreren, Grundelementen und mindestens einem, bevorzugt mehreren, Strömungsleitelementen. Dabei bildet mindestens je ein Strömungsleitelemente von zumindest zwei, bevorzugt mehreren, Segmenten eine erste Gruppe von Strömungsleitelementen. Diese Strömungsleitelemente der ersten Gruppe sind so angeordnet, dass sie vom ersten Ende des Grundkörpers über die ringförmigen Segmente zum zweiten Ende des Grundkörpers dem Verlauf einer dreidimensionalen Schraube folgen. Gleiches gilt für die weiteren Gruppen von Strömungsleitelementen.

Bevorzugt sind dabei die Grundelemente und die Strömungsleitelemente in einem mäanderförmigen Muster, insbesondere einem Zick-Zack-Muster, angeordnet. In dieser Ausgestaltung der Erfindung, wo Grundelemente und Strömungsleitelemente in einem Zick-Zack Muster angeordnet werden, folgt innerhalb eines ringförmigen Segmentes jeweils ein Grundelement auf ein Strömungsleitelement. Ebenso zweckmäßig sind andere mäanderförmige Anordnungen, wo immer Grundelement und Strömungsleitelement abwechseln. Als Strömungsleitelemente fungieren dabei die Teile des Mäanders, die Anteile mit einem Winkel zur Längsachse des Grundkörpers aufweisen und insgesamt über den gesamten Grundkörper einen schraubenförmigen Verlauf folgen.

Vorteilhafterweise weisen die Strömungsleitelemente eine größere Höhe als die Grundelemente und/oder Verbinder auf. In dieser Ausgestaltung der Erfindung sorgt die größere Höhe der Strömungsleitelemente dafür, dass die Strömungsleitelemente die Strömung führen und die Grundelemente und/oder Verbinder keinen oder nur einen vernachlässigbaren strömungsführenden Einfluss ausüben.

Unter der Höhe eines Elements oder eines Verbinders wird im Rahmen dieser Erfindung die Ausdehnung der Elemente oder Verbinder in radialer Richtung verstanden.

Durch die größere Höhe wirken die Strömungsleitelement auf die anströmende Flüssigkeit ähnlich wie ein Wehr. Bei entsprechender Orientierung der Strömungsleitelemente lässt sich die Strömung entsprechend an den Strömungsleitelementen entlang führen. Dies sorgt für die erfindungsgemäße Führung der Strömung in Form einer Schraube.

Bevorzugt bildet die Höhe der Strömungsleitelemente des Grundgerüsts zur Höhe der Grundelemente und/oder Verbinder ein Verhältnis mehr als 1.5 : 1, bevorzugt mehr als 2:1. Insbesondere haben die Strömungsleitelemente eine Höhe von mehr als 100 µm, bevorzugt mehr als 200 µm. Dabei werden Strömungsleitelemente mit einer Höhe ab 100 µm zweckmäßigerweise bei Stents im Bereich der koronaren Anwendung eingesetzt. Strömungsleitelemente mit einer Höhe von mehr als 200 µm sind bei peripheren Anwendung vorteilhaft.

In einer anderen Ausgestaltung der Erfindung weisen die Strömungsleitelemente eine Vertiefung auf der luminalen Seite auf.

Die Endoprothese wird bei Implantation gegen die Gefäßwand gepresst oder gedrückt. Die Seite des Elements des Grundgerüstes, welche die äußere Mantelseite der zylinderförmigen Endoprothese bildet, wird gegen die Gefäßwand gedrückt. Diese Seite wird als abluminale Seite oder als abluminaler Anteil des Elements bezeichnet. Die anderen Seiten des Querschnitts des Elements sind entsprechend im Lumen des Köpergefäßes ohne Kontakt mit der Gefäßwand und werden als luminale Seite oder als luminaler Anteil des Elements bezeichnet.

In dieser Ausgestaltung der Erfindung weisen die Strömungsleitelemente zumindest eine Vertiefung auf einer luminalen Seite auf. Bevorzugt haben die Elemente des Grundgerüstes einen rechteckigen Querschnitt. Die Vertiefung wird in dieser Ausgestaltung der Erfindung bevorzugt auf der luminalen Seite des Querschnitts der Strömungsleitelemente angebracht, die der abluminalen Seite direkt gegenüberliegt. In dieser Ausgestaltung der Erfindung verursacht die Vertiefung in den Strömungsleitelementen Reflektionen bzw. werden Anteile der Strömung rezirkuliert. Dadurch wirkt die Vertiefung ähnlich einem Wehr und die Strömung wird entlang der Vertiefung geführt.

Diese Ausgestaltung der Erfindung kann mit den vorangehend und folgend beschriebenen Ausgestaltungen kombiniert werden.

Bevorzugt entspricht die Bogenlänge aller Strömungsleitelemente eines ringförmigen Segmentes mindestens dem halben Umfang des zylindrischen Grundkörpers. Um einen hinreichenden Einfluss auf die Strömung ausüben, muss eine hinreichende Fläche durch die Strömungsleitelemente in der Strömung vorhanden sein, um die Strömung zu führen. Dies wird in dieser bevorzugten Ausgestaltung der Erfindung dadurch erreicht, dass die Bogenlänge aller Strömungsleitelemente zusammen mindestens den halben, bevorzugt mehr als dem halben, Umfang des zylindrischen Grundkörpers entspricht. Zweckmäßigerweise muss die Bogenlänge aller Strömungsleitelemente eines Segmentes entsprechend länger sein, je kleiner die Höhe der Strömungsleitelemente ist.

Unter der Bogenlänge wird dabei die Länge des dreidimensionalen Bogens verstanden, den das Strömungsleitelement auf der Mantelfläche des zylindrischen Grundkörpers beschreibt. In einer anderen Ausgestaltung der Erfindung besteht das Grundgerüst aus zumindest zwei Elementen, die die dreidimensionale Form einer Schraube aufweisen, wobei Radius, Steigung und Gängigkeit der Schraube für beide Elemente gleich sind und die beiden Elemente über zumindest einen Verbinder verbunden sind, wobei der/die Verbinder eine geringere Höhe als die Elemente aufweisen.

In dieser Ausgestaltung der Erfindung erfolgt die Führung der Strömung durch die Form des Grundgerüstes selbst. Das Grundgerüst dieser Ausgestaltung der Erfindung besteht aus mindestens zwei schraubenförmigen Elementen. Die Elemente des Grundgerüstes werden durch Verbinder mit geringerer Höhe als die Elemente verbunden. Die Strömung durch die Endoprothese vom ersten Ende zum zweiten Ende wird in dieser Ausgestaltung der Erfindung durch das Grundgerüst selbst geführt. Die Elemente des Grundgerüstes wirken ähnlich wie ein Wehr auf die Strömung. Die Strömung strömt zumindest anteilig entlang des Wehres, wodurch insbesondere die wandnahe Strömung in eine Schraubenform geführt wird. Die geringere Höhe der Verbinder stellt sicher, dass die Verbinder keinen oder nur einen vernachlässigbaren Einfluss auf die Strömung haben.

Bevorzugt ist/sind der/die Verbinder als ein flächiges Element ausgestaltet und auf der abluminalen Seite des Grundgerüsts angeordnet. Ebenso zweckmäßig ist/sind der/die Verbinder als Steg ausgestaltet und zwischen den Elementen angeordnet. Eine Kombination dieser beiden Ausgestaltungen kann für bestimmte Ausführungsformen der Erfindung ebenfalls zweckmäßig sein.

In einer weiteren Ausgestaltung weist zumindest ein Element des Grundgerüsts auf der luminalen Seite Ausformungen auf, die zur Beeinflussung des Strömung geeignet sind, wobei die Ausformungen bevorzugt durch eine Beschichtung des/der Elements/Elemente gebildet wird. In dieser Ausgestaltung der Erfindung werden bevorzugt mehrere Elemente des Grundgerüstes mit Ausformungen zur Beeinflussung der Strömung versehen. Die Form derartiger Ausformungen kann vielfältig und auf die jeweiligen Strömungsverhältnisse abgestimmt sein. Vorteilhaft sind hier beispielsweise Ausformungen, die Profilen entsprechen, wie sie in der NACA-Liste hinterlegt sind. Eine derartige Form wird leicht umströmt, wodurch die Strömung insgesamt geführt werden kann. Fertigungstechnisch lässt sich eine derartige Ausformung am einfachsten über eine entsprechende Beschichtung der/des Elements des Grundgerüstes erreichen. Die Ausformung wird dabei am zweckmäßigsten auf der luminalen Seite gegenüber der abluminalen Seite des entsprechenden Elements platziert. Diese Ausgestaltung der Erfindung kann mit allen vorangehend geschilderten Ausgestaltungen kombiniert werden.

Unter einer Ausformung wird im Rahmen dieser Ausgestaltung eine dreidimensionale Struktur verstanden, die sich auf der Grundform des Elementes befindet. Die Ausformung und das Element können aus einem Materialstück sein oder die Ausformung wird bevorzugt durch eine Beschichtung gebildet.

Zweckmäßigerweise bestehen das Grundgerüst (die einzelnen Elemente und Verbinder) aus biokompatiblem Metall, insbesondere einer Cobalt-Basis-Legierung, bevorzugt einem biologisch degradierbaren Metall, insbesondere einer Magnesium Legierung, oder einem Formgedächtnismaterial, insbesondere Nitinol, oder einem Polymer. In einigen Ausgestaltungen der Erfindung ist es jedoch zweckmäßig die Strömungsleitelemente aus einem anderen Material als die restlichen Elemente des Grundgerüstes zu fertigen.

Durch die vorliegende Erfindung gelingt es, eine intraluminale Endoprothese zur Verfügung zu stellen, in der bei Durchströmung vom ersten zum zweiten Ende nahezu natürliche Strömungsverhältnisse herrschen. Dies ist insbesondere bei einem Stent zur Implantation in einem arteriellen Blutgefäß der Fall, wodurch das Risiko der Restenose deutlich gegenüber dem Stand der Technik gesenkt wird.

Im Folgenden soll die Erfindung anhand der in den Figuren dargestellten Ausführungsbeispiele näher erläutert werden.

Es zeigen:
- Fig. 1: ein Ausführungsbeispiel der Erfindung mit einem Grundgerüst aus mehreren Ringsegmenten in Reihenanordnung,
- Fig. 2: ein Ausführungsbeispiel der Erfindung mit einem Grundgerüst aus mehreren Ringsegmenten in gespiegelter Anordnung,
- Fig. 3: den Querschnitt eines Grundelements und eines Strömungsleitelements nach den Ausführungsbeispielen aus Fig. 1 und Fig. 2,
- Fig. 4: den Querschnitt eines alternativen Strömungsleitelements nach den Ausführungsbeispielen aus Fig. 1 und Fig. 2,
- Fig. 5: ein Ausführungsbeispiel der Erfindung mit einem Grundgerüst aus Elementen in Form einer Schraube und
- Fig. 6: ein Ausführungsbeispiel eines Elementes mit einer Ausformung.

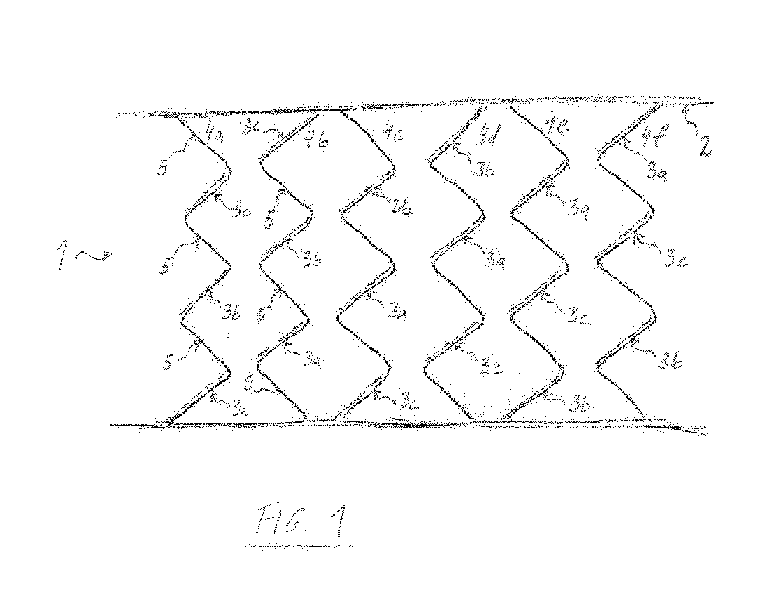

Figur 1 zeigt einen Ausschnitt aus eines Ausführungsbeispiels einer erfindungsgemäßen Endoprothese als Stent 1 in ein Körpergefäß 2 implantiert. Der Stent 1 wird dabei von seinem ersten Ende zu seinem zweiten Ende durchströmt (in den Figuren von links nach rechts).

Der Stent 1 in Figur 1 weist in diesem Ausführungsbeispiel ein Grundgerüst aus, welches aus 6 ringförmigen Segmenten 4a, 4b, 4c, 4d, 4e und 4f besteht. Die Ringsegmente 4a, 4b, 4c, 4d, 4e und 4f sind mäanderförmig und bestehen aus den im Zick-Zack-Muster angeordneten Grundelementen 5 und den Strömungsleitelementen 3a, 3b und 3c. Die Ringsegmente 4a, 4b, 4c, 4d, 4e und 4f sind dabei jeweils über mehrere Verbinder (nicht dargestellt) miteinander verbunden. Die einzelnen Ringsegmente sind dabei jeweils gespiegelt angeordnet. Das heißt in Richtung der Längsachse des Stents 1 folgt auf ein Maximum des Ringsegments 4a ein Minimum des Ringsegmentes 4b und so weiter.

Der Stent 1 weist mehrere Gruppen von Strömungsleitelementen (in der Figur 1 durch Doppelstriche dargestellt) auf, wobei 3 Gruppen von Strömungsleitelementen in Fig. 1 dargestellt sind. Dabei bilden jeweils die Strömungsleitelemente 3a, die Strömungsleitelemente 3b und die Strömungsleitelemente 3c eine Gruppe von Strömungsleitelementen. Die Strömungsleitelemente 3a, 3b und 3c jeder Gruppe sind dabei so angeordnet, dass sie jeweils den dreidimensionalen Verlauf einer Schraube folgen. Die Gruppen der Strömungsleitelemente 3a, 3b und 3c sowie einzelne Strömungsleitelemente beschreiben eine Schar von schraubenförmigen Kurven. Die Kurvenscharen unterscheiden sich dabei lediglich in ihrer Phase. Weitere Merkmale wie Radius, Steigung und Gängigkeit sind bei jeder schraubenförmigen Kurve dieser Schar konstant.

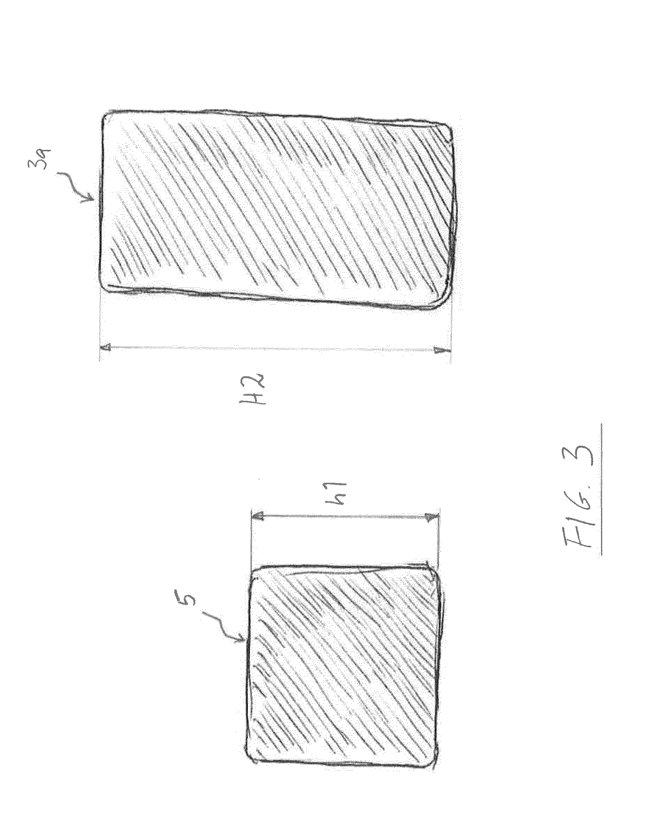

In Figur 3 ist exemplarisch der Querschnitt eines Grundelementes 5 und eines Strömungsleitelementes 3a dargestellt. Die Höhe H2 der Strömungsleitelemente 3a, 3b und 3c ist den Ausführungsbeispiel nach Figur 1 doppelt so groß wie die Höhe h1 des Grundelementes 5 und der Verbinder (nicht dargestellt).

Durch die größere Höhe der Strömungsleitelemente 3a, 3b und 3c wird die Führung der Strömung durch den Stent 1 (von links nach rechts in der Figur) gewährleistet. Die von links strömende Flüssigkeit strömt gegen die Strömungsleitelemente 3a, 3b und 3c und wird an diesen entlang geführt. Durch die Vielzahl der in Gruppen angeordneten Strömungsleitelemente 3a, 3b und 3c, die jeweils dem dreidimensionalen Verlauf einer Schraube mit gleichem Radius, gleicher Steigung und gleicher Gängigkeit folgen, wird die Strömung insgesamt schraubenförmig geführt. Die Grundelemente 5 und die Verbinder (nicht dargestellt) tragen aufgrund ihrer geringeren Höhe nicht oder nur vernachlässigbar zum Strömungsverhalten der Flüssigkeit im Stent 1 bei.

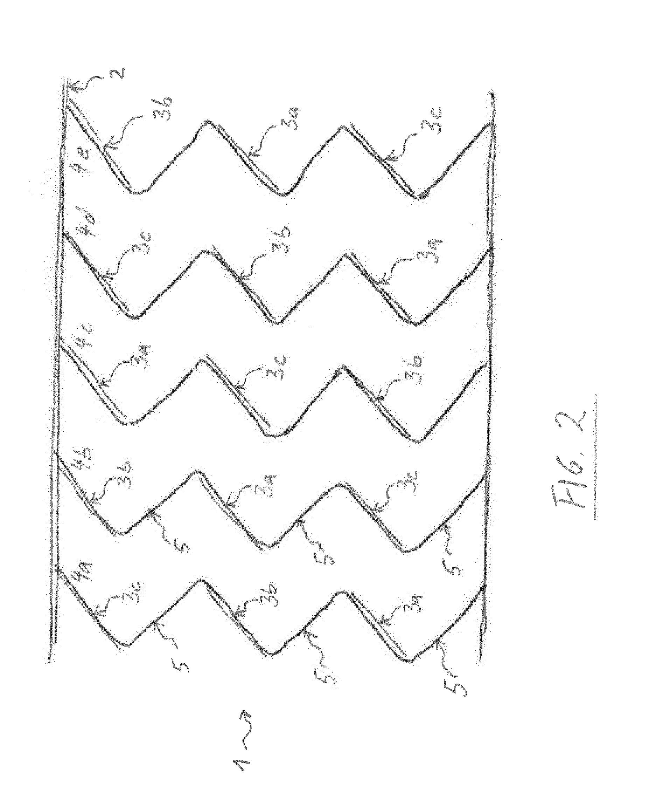

Das in Figur 2 dargestellte Ausführungsbeispiel des Stents 1 unterscheidet sich vom Ausführungsbeispiel nach Figur 1 nur dadurch, dass die ringförmigen Segmente 4a, 4b, 4c, 4d und 4e hier in Reihe angeordnet sind. Auf ein Maximum des ringförmigen Segmentes 4a folgt in Richtung der Längsachse (von links nach rechts in der Figur) ein Maximum des benachbarten ringförmigen Segmentes 4b und so weiter.

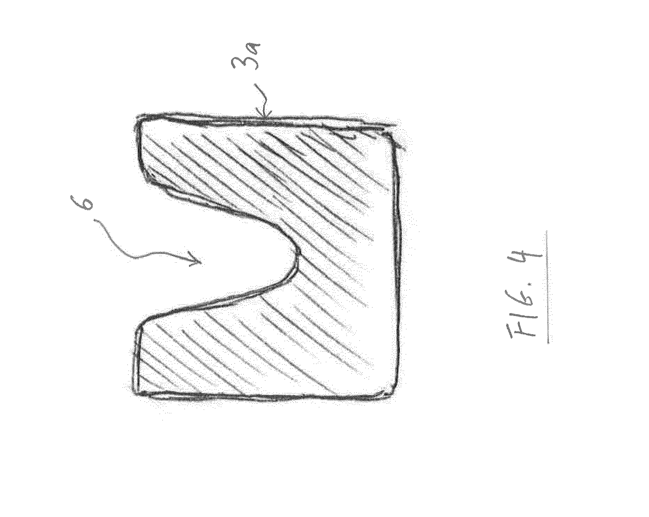

Figur 4 zeigt den Querschnitt einer Ausgestaltung des Strömungsleitelementes 3a, wie es als Strömungsleitelemente 3a, 3b und 3c in den Ausführungsbeispielen der Figuren 1 und 2 alternativ eingesetzt werden kann. In dieser Ausgestaltung weist das Strömungsleitelement 3a eine Aussparung 6 in Form einer tiefen Kerbe oder Rille auf. Wie vorangehend geschildert, funktioniert eine Kerbe ähnlich einem Wehr. Das Strömungsleitelement 3a nach Figur 4 kann daher auch für jedes der Strömungsleitelemente 3a, 3b und 3c der Figuren 1 und 2 verwendet werden. Denkbar sind auch Kombinationen wie beispielsweise Strömungsleitelemente 3a nach Figur 4 und Strömungsleitelemente 3b und 3c nach Figur 3. Ebenfalls ist es möglich, bei der Verwendung von Strömungsleitelementen 3a nach Figur 4, diese mit der gleichen Höhe h1 wie die Grundelemente 5 und die Verbinder (nicht dargestellt) oder mit der doppelten Höhe H2 auszuführen.

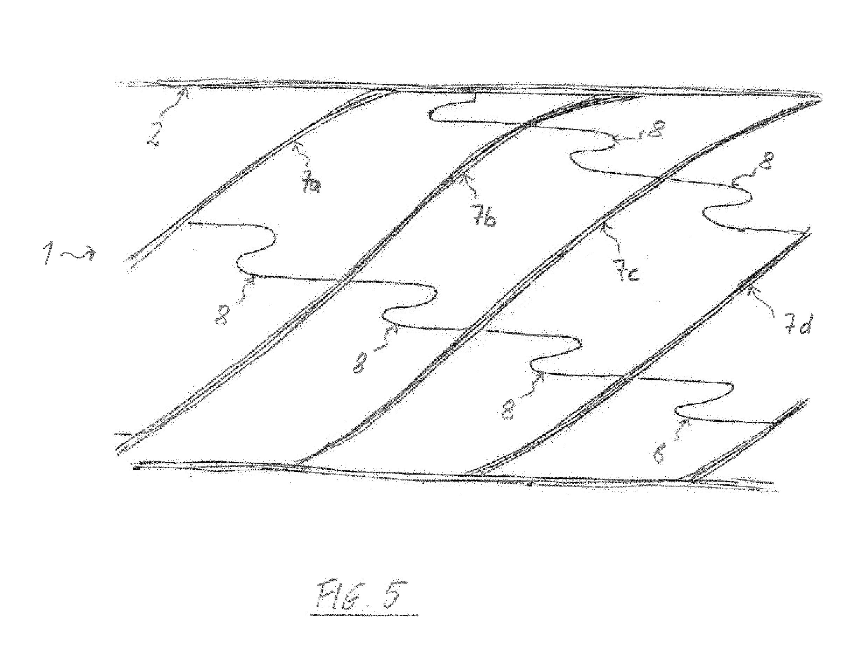

Figur 5 zeigt eine alternative Ausgestaltung der erfindungsgemäßen Endoprothese als Stent 1 (nur ausschnittsweise dargestellt). In dieser Ausgestaltung der Erfindung besteht das Grundgerüst des Stents 1 aus mehreren schraubenförmigen Elementen 7a, 7b, 7c und 7d, die über Verbinder 8 miteinander verbunden sind. Die schraubenförmigen Elemente 7a, 7b, 7c und 7d weisen dabei eine größere Höhe (in dieser Ausgestaltung die doppelte Höhe) als die Verbinder 8 aus. In diesem Ausführungsbeispiel der Erfindung wird die Strömung durch das Grundgerüst selbst geführt. Die größere Höhe der Elemente 7a, 7b, 7c und 7d stellt sicher, das die Strömung durch die Elemente 7a, 7b, 7c und 7d geführt wird und die Verbinder 8 das Strömungsverhalten nicht oder nur vernachlässigbar beeinflussen.

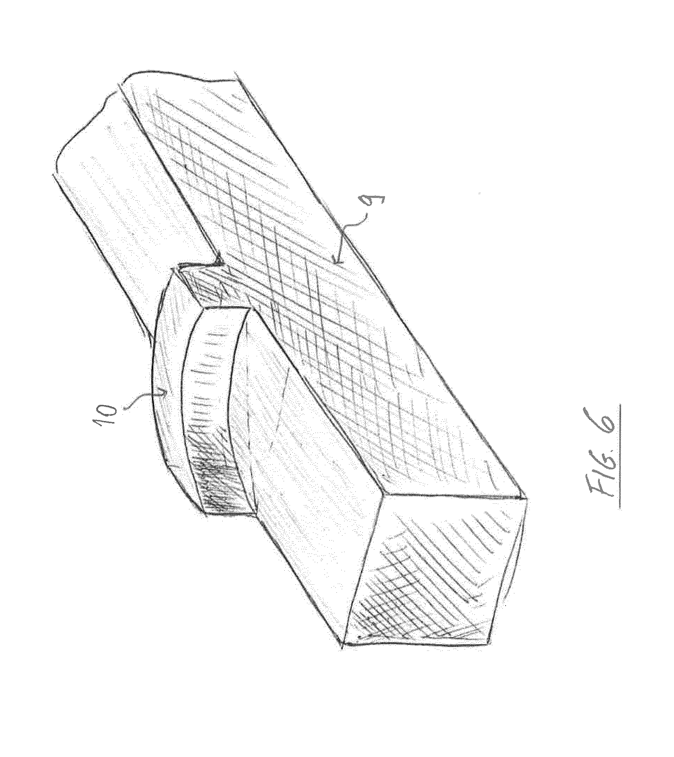

Figur 6 zeigt ein beliebiges Element 9 eines Grundgerüsts eines Stents 1 mit einer Ausformung 10 zur Führung der Strömung. In diesem Ausführungsbeispiel eines Elementes 7 ist auf der luminalen Seite eine Ausformung 10 zur Strömungsführung angebracht. Die Ausformung 10 wird über eine Beschichtung (beispielsweise eine Polymerbeschichtung) auf der luminalen Seite des Elementes 9 angebracht. Diese Ausgestaltung kann mit allen in den Figuren gezeigten Ausführungsbeispielen kombiniert werden.

## Patentansprüche

1. Intraluminale Endoprothese (1) geeignet zur Implantation in einem durchströmten Körpergefäß (2) und aufweisend einen zylindrischen Grundkörper mit einem ersten Ende und einem zweiten Ende, wobei der Grundkörper aus einem Grundgerüst von zumindest zwei einzelnen, miteinander verbundenen Elementen (3a, 3b, 3c, 5, 7a, 7b, 7c, 7d, 8, 9) besteht, und das Grundgerüst geeignet ist, eine mechanische Stützwirkung im Körpergefäß (2) zu erzielen,
**dadurch gekennzeichnet, dass**
das Grundgerüst Mittel (3a, 3b, 3c, 7a, 7b, 7c, 7d, 10) aufweist, die zur Strömungsführung innerhalb des Grundkörpers geeignet sind, wenn der Grundkörper von seinem ersten Ende zu seinem zweiten Ende durchströmt wird, und die Mittel (3a, 3b, 3c, 7a, 7b, 7c, 7d, 10) derart ausgestaltet sind, dass die Strömung innerhalb des Grundkörpers als Schraube geführt wird.

2. Endoprothese (1) nach Anspruch 1, **dadurch gekennzeichnet, dass** das Grundgerüst aus zumindest zwei Grundelementen (5) und zumindest zwei Strömungsleitelementen (3a, 3b, 3c) besteht, wobei zumindest eine erste Gruppe (3a, 3b, 3c) von zumindest zwei Strömungsleitelementen (3a, 3b, 3c) derart angeordnet ist, dass die Strömungsleitelemente (3a, 3b, 3c) der der ersten Gruppe einer dreidimensionalen Kurve in Form einer Schraube folgen.

3. Endoprothese (1) nach Anspruch 2, **dadurch gekennzeichnet, dass** das Grundgerüst mehrere Gruppen von Strömungsleitelementen (3a, 3b, 3c) aufweist, wobei die Strömungsleitelemente (3a, 3b, 3c) in jeder Gruppe so angeordnet sind, dass sie einer dreidimensionalen Kurve in Form einer Schraube folgen, wobei Radius, Steigung und Gängigkeit der Schraube für jede Gruppe von Strömungsleitelementen (3a, 3b, 3c) identisch ist.

4. Endoprothese nach Anspruch 2 oder 3, **dadurch gekennzeichnet, dass** das Grundgerüst aus mehreren ringförmigen Segmenten (4a, 4b, 4c, 4d, 4e, 4f) besteht, wobei jedes Segment (4a, 4b, 4c, 4d, 4e, 4f) aus mindestens einem Grundelement (5) und mindestens einem Strömungsleitelement (3a, 3b, 3c) besteht, und die Segmente (4a, 4b, 4c, 4d, 4e, 4f) über mindestens einen Verbinder miteinander verbunden sind.

5. Endoprothese (1) nach Anspruch 4, **dadurch gekennzeichnet, dass** das Grundgerüst mindestens zwei ringförmige Segmente (4a, 4b, 4c, 4d, 4e, 4f) aufweist, die parallel zueinander oder gespiegelt zueinander angeordnet sind.

6. Endoprothese (1) nach einem der Ansprüche 2 bis 5, **dadurch gekennzeichnet, dass** die Strömungsleitelemente (3a, 3b, 3c) eine größere Höhe als die Grundelemente (5) und/oder Verbinder aufweisen.

7. Endoprothese (1) nach einem der Ansprüche 2 bis 6, **dadurch gekennzeichnet, dass** die Strömungsleitelemente (3a, 3b, 3c) eine Vertiefung auf der luminalen Seite aufweisen.

8. Endoprothese (1) nach einem der Ansprüche 2 bis 7, **dadurch gekennzeichnet, dass** die Bogenlänge aller Strömungsleitelemente (3a, 3b, 3c) eines ringförmigen Segmentes (4a, 4b, 4c, 4d, 4e, 4f) mindestens dem halben Umfang des zylindrischen Grundkörpers entspricht.

9. Endoprothese (1) nach Anspruch 1, **dadurch gekennzeichnet, dass** das Grundgerüst aus zumindest zwei Elementen (7a, 7b, 7c, 7d) besteht, die die dreidimensionale Form einer Schraube aufweisen, wobei die Radius, Steigung und Gängigkeit der Schraube für beide Elemente (7a, 7b, 7c, 7d) gleich sind und die beiden Elemente (7a, 7b, 7c, 7d) über zumindest einen Verbinder (8) verbunden sind, wobei der/die Verbinder (8) eine geringere Höhe als die Elemente aufweisen.

10. Endoprothese (1) nach Anspruch 9, **dadurch gekennzeichnet**, der/die Verbinder (8) als ein flächiges Element ausgestaltet und auf der abluminalen Seite des Grundgerüsts angeordnet ist.

11. Endoprothese (1) nach Anspruch 9 oder 10, **dadurch gekennzeichnet, dass** der/die Verbinder (8) als Steg ausgestaltet und zwischen den Elementen (7a, 7b, 7c, 7d) angeordnet ist.

12. Endoprothese (1) nach einem der vorangegangenen Ansprüche, **dadurch gekennzeichnet, dass** zumindest ein Element (3a, 3b, 3c, 5, 7a, 7b, 7c, 7d, 8, 9) des Grundgerüsts auf der luminalen Seite Ausformungen (10) aufweist, die zur Beeinflussung des Strömung geeignet sind, wobei die Ausformungen (10) bevorzugt durch eine Beschichtung des/der Elements/Elemente (3a, 3b, 3c, 5, 7a, 7b, 7c, 7d, 8, 9) gebildet wird.
